# EUROPEAN PATENT SPECIFICATION

(11) **EP 1 677 109 B1**
(45) Date of publication and mention of the grant of the patent: **11.07.2007**
(21) Application number: 04107052.5
(22) Date of filing: 29.12.2004
(51) Int. Cl.: G01N 33/18, G01N 27/06

(54) **Contactless water conductivity measuring device**
Vorrichtung zum berührungsfreien messen der Leitfähigkeit von Wasser
Dispositif de mesure sans contact de la conductivité de l'eau

(43) Date of publication of application: 05.07.2006
(73) Proprietor: Electrolux Home Products Corporation N.V., 1930 Zaventem (BE)
(72) Inventor: Uhov, Andrei, St-Petersburg (RU); Cenedese, Claudio, 33037, Pasian di Prato (IT); Kolgin, Evgenij, St-Petersburg (RU)
(74) Representative: Giugni, Valter

(56) References cited:
- EP-A- 1 010 976
- DE-A1- 4 216 176
- DE-A1- 19 841 568
- US-A- 5 017 879
- US-A- 5 266 899
- US-A1- 2002 067 174
- US-A1- 2003 222 664

## Description

The present invention refers to a new contactless water conductivity measuring device, which is employed in appliances like washing machines and similar.

As it is known, conductivity is normally measured by using two metal electrodes by means of which a voltage is applied, while the current flowing through a solution, as generated by such voltage application, is at the same time measured by means of said electrodes. However, when the electrodes are plunged in the solution, serious drawbacks occur like corrosion of electrodes, oxidation, electrolysis and potential electrical hazard for eventual circuit faults.

EP 1010976 discloses a non-contact measuring device for measuring the specific conductivity of electrolytic solutions. The device comprises two coupling electrodes, each consisting of a good conducting substrate on whose sensor side a very thin layer of ceramic dielectric is applied. This means that the ceramic layer works as an electric insulation and as a protection against the liquid being measured. Indeed, the device may be also used as an immersion sensor.

The described electrode is complex to manufacture and the process to control the correct insulation is critical too. Moreover, the measurement is carried out at a frequency in the low frequency range, wherein the flowing electric current is assessed on the basis of the voltage drop at a measuring resistance. Therefore, the device sensibility is poor.

US 5,017,879 discloses an apparatus for use in the capacitive measurement of the void fraction in a flowing liquid. An assembly of at least two electrodes is spaced apart around the exterior of the body defining a flow passage such that the capacitance of the electrode assembly is a function of the dielectric constant of the material within the passageway. Apart from the different technical field of application, this apparatus teaches to use electrodes whose surfaces are diametrically faced each other with respect to the longitudinal axis of the flow passage. Such an assembly does not allow an easy regulation of the distance between two electrodes, so as it is quite difficult to set the apparatus sensibility.

US 2003/222664 A1 discloses a device and method for contactless heating of a fluid in a chamber using a high frequency alternating potential. At least two electrodes are coupled to the chamber such that the alternating potential is coupled to the fluid inside the chamber and heats the fluid. The device and method described determine temperature and/or conductivity of the fluid. However, no valuable information for the functioning of an associated apparatus are derived from the conductivity measurement.

The scope of the present invention is defined by the device as defined in claim 1 and in the subsequent dependent claim.

Features and advantages of the present invention will be clearly apparent from the following description, given as non limiting example, with reference to the annexed drawings, wherein:
- Figure 1 shows a schematic view of a first embodiment of the present invention;
- Figure 2 shows a schematic view of a second embodiment of the present invention;
- Figure 3 shows a functional block diagram of a contactless water conductivity measuring device according to the present invention.

The basic principle of a contactless water measuring device according the present invention is to measure the capacitive current flowing through the water at high frequency.

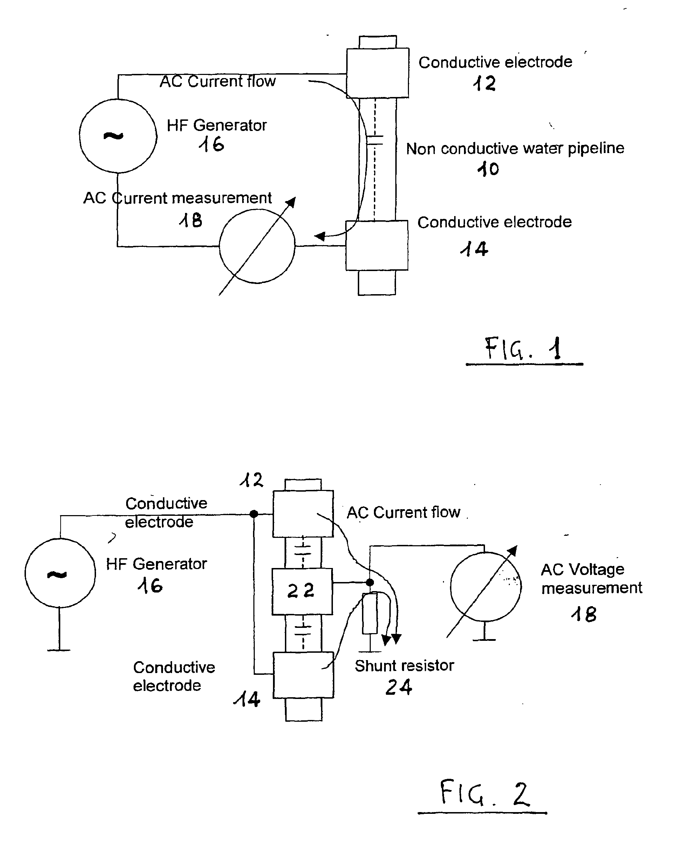

A first embodiment of the device is shown in Figure 1, wherein in a pipeline 10, made of a non conductive material, water or a similar fluid flows and its conductivity should be measured in order to obtain valuable information for the functioning of an associated apparatus. For instance, in a washing machine or a dishwasher it is possible to check the presence of detergent in the washing water and, consequently, to set the rinsing cycle appropriately. Another example is represented by the use of a device according to the present invention in an ice-maker to detect the quantity of salts within the inlet water.

As shown in Figure 1, two electrodes 12 and 14, each made in the shape of a flat metallic strip, are wrapped around the external surface of the pipeline 10, spaced apart each other along the longitudinal axis of the pipeline. Preferably, copper foil strips are used as electrodes. However, copper wires are also usable; in this case each electrodes should be done by few turns over pipeline (about 10 mm. width).

As a result, the pipeline and the electrodes become a capacitor. High frequency (few MHz) is applied to the capacitor by means of an electric circuit comprising an HF generator 16 and is measured by an AC measuring apparatus (ammeter) 18. Such a circuit is a direct current measurement circuit and the electric current will depend from the pipeline characteristics (size and material) and from water conductivity.

Figure 2 shows a second embodiment of the device according to the invention. This embodiment utilises three electrodes 12, 14, 22 and measure the voltage drop on a shunt resistor 24 by means of an AC voltage measurement apparatus (voltmeter) 26. The shunt resistor 24 is connected to the third electrode 22. Voltage drops on the resistor 24 are proportional to the current through the pipeline 10.

In each embodiment an electronic board (not represented), to which the electrodes are connected, controls the signals and the measuring system. The electronic board is of known kind and is adjusted following the different type of apparatus to which the device has to be applied.

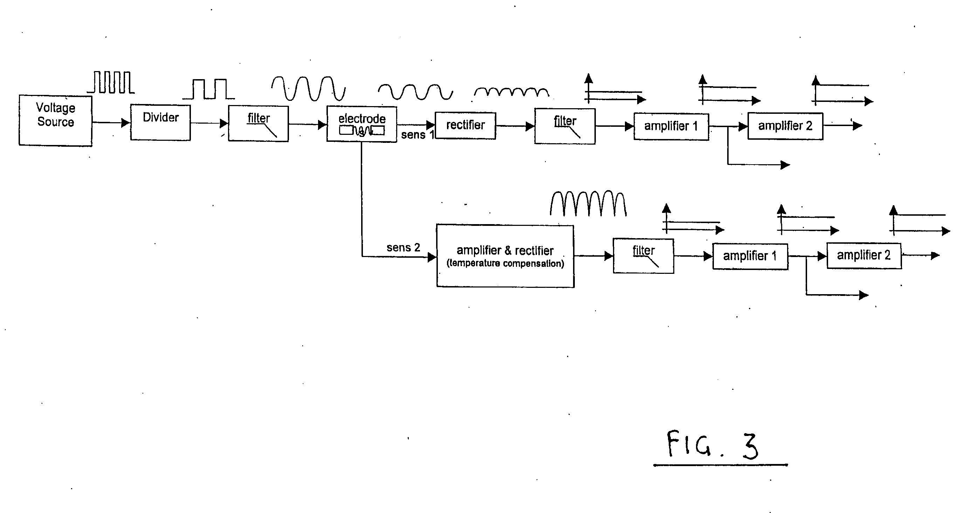

Figure 3 shows a functional block diagram of a device according to the present invention. The diagram shows a first part (comprising a voltage source, a frequency divider and a filter) which is common to the different embodiments of Figure 1 and Figure 2. Then, starting from the electrode, the diagrams branches out in two ways corresponding to the two embodiments referred to in Figure 1 and Figure 2, respectively. Of course, the two different embodiments are alternative each other and the choice of the right one has to be made according to the convenience.

The advantages of a device as described are quite evident.

Electrodes are insulated and kept physically away from the environment in which the measured specimen is contained. The layout avoids common problems of conductivity measurements with immersed electrodes as: corrosion, oxidation, chemical reaction on their surface, scale and/or contaminant deposition. These kinds of phenomena are known to be the major causes of reduction of linearity, repeatability and lifetime of common sensors because they modify the sensing unit characteristics thus impairing measurements.

Safety is increased since electrical components are sealed and not in contact with water or any other liquid subject to measurement.

Chosen measurement frequency grants optimal performances for water measurements since it is tuned regarding the ion species normally present in water.

The proposed electrodes arrangement allows having excellent measurement cell when installed on pipes, if compared to common facing shields layout. The measurement cell results bigger than usual, its dimensions can be easily adjusted by sliding electrodes along the piping for matching the optimal sensitivity, shielding is extremely convenient and the electric field path through the measured fluid is increased and well controlled.

Knowing the statistical relation between hardness and conductivity in household water distribution system one can also say that the sensor can be used with good confidence to perform water hardness measurements.

## Claims

1. A washing machine or similar household apparatus provided with a contactless water conductivity measuring device comprising at least two conducting electrodes which are connected in an electric circuit comprising an HF generator and are applied to the external surface of a non-conductive pipeline wherein water flows, the conducting electrodes (12,14) each consist of a conductive strip wrapped around the pipeline (10) and are spaced apart from each other along the longitudinal axis of the pipeline, **characterised in that** the electric circuit to measure the water conductivity comprises an AC measurement apparatus (18).

2. A washing machine or similar household apparatus provided with a contactless water conductivity measuring device according to claim 1, comprising three conducting electrodes, **characterised in that** the electric circuit to measure the water conductivity comprises an AC voltage measurement apparatus (24) which is connected to the third electrode (22) by means of a shunt resistor (26).

## Patentansprüche

1. Waschmaschine oder ein ähnliches Haushaltsgerät, versehen mit einer kontaktlosen Vorrichtung zum Messen der Wasserleitfähigkeit, die mindestens zwei leitende Elektroden umfasst, die in einem elektrischen Schaltkreis verbunden sind, der einen HF-Generator umfasst, und an die Außenoberfläche einer nicht leitenden Rohrleitung angelegt sind, in der Wasser fließt, wobei die leitenden Elektroden (12, 14) jeweils aus einem leitenden Streifen bestehen, der um die Rohrleitung (10) gewickelt ist, und voneinander an der Längsachse der Rohrleitung beabstandet sind, **dadurch gekennzeichnet, dass** der elektrische Schaltkreis zum Messen der Wasserleitfähigkeit ein Wechselstrommessgerät (18) umfasst.

2. Waschmaschine oder ein ähnliches Haushaltsgerät, versehen mit einer kontaktlosen Vorrichtung zum Messen der Wasserleitfähigkeit gemäß Anspruch 1, die drei leitende Elektroden umfasst, **dadurch gekennzeichnet, dass** der elektrische Schaltkreis zum Messen der Wasserleitfähigkeit ein Wechselstrom-Spannungsmessgerät (24) umfasst, das an der dritten Elektrode (22) mittels eines Nebenwiderstands (26) angeschlossen ist.

## Revendications

1. Une machine à laver ou un appareil ménager similaire pourvu d'un dispositif de mesure sans contact de la conductivité de l'eau, comprenant au moins deux électrodes conductrices qui sont connectées à un circuit électrique comprenant un générateur à haute fréquence (HF) et qui sont appliquées sur la surface extérieure d'un conduit d'écoulement d'eau non conducteur, chaque électrode conductrice (12, 14) consistant en un ruban conducteur enroulé autour du conduit (10) et étant disposées séparément l'une de l'autre le long de l'axe longitudinal du dit conduit, **caractérisé en ce que** le circuit électrique de mesure de la conductivité de l'eau comporte un appareil de mesure du courant alternatif (18).

2. Une machine à laver ou un appareil ménager similaire pourvu d'un dispositif de mesure sans contact de la conductivité de l'eau selon la revendication 1, comprenant trois électrodes conductrices, **caractérisé en ce que** le circuit électrique de mesure de la conductivité de l'eau comporte un appareil de mesure de la tension du courant alternatif (24), connecté à la troisième électrode (22) au moyen d'une résistance de dérivation (26).
